# EUROPEAN PATENT APPLICATION

(11) **EP 4 101 475 A1**
(43) Date of publication of application: **14.12.2022**
(21) Application number: 22177092.8
(22) Date of filing: 02.06.2022
(51) Int. Cl.: A61L 2/10, A61L 9/20

(54) **SYSTEMS AND METHODS FOR SANITIZING PORTIONS OF AN ENCLOSED SPACE AND ALLOCATING SANITIZING RESOURCES WITHIN THE ENCLOSED SPACE**

(30) Priority: 07.06.2021 US 202163197616 P
(71) Applicant: The Boeing Company, Chicago, IL 60606-1596 (US)
(72) Inventor: DACZKO, Mateus S., CHICAGO, 60606-1596 (US); SHAH, Neil N., CHICAGO, 60606-1596 (US)
(74) Representative: Arnold & Siedsma

(57) **Abstract**

A system (100) for sanitizing an enclosed space (104) includes one or more sensors (120) within the enclosed space (104). The one or more sensors (120) are configured to detect one or more actions within the enclosed space (104) and output one or more action detection signals (130) indicative of the one or more actions. One or more ultraviolet (UV) lamps (110) are within the enclosed space (104). The one or more UV lamps (110) are configured to emit UV light (112) in relation to one or more portions of the enclosed space (104). A sanitizing control unit (116) is in communication with the one or more sensors (120) and the one or more UV lamps (110). The sanitizing control unit (116) is configured to receive the one or more action detection signals (130) from the one or more sensors (120). The sanitizing control unit (116) is configured to modify operation of the one or more UV lamps (110) in response to the one or more actions being a triggering event. Additionally, or optionally, a resource allocation control unit (132) is configured to generate resource recommendations regarding the one or more ultraviolet (UV) lamps (110) for the enclosed space (104) based on one or more triggering events of the one or more actions.

## Description

### FIELD OF THE DISCLOSURE

Examples of the present disclosure generally relate to systems and methods for sanitizing portions (such as fluid and surfaces within) of an enclosed space, such an internal cabin of a commercial aircraft, and allocating sanitizing resources within the enclosed space.

### BACKGROUND OF THE DISCLOSURE

Vehicles such as commercial aircraft are used to transport passengers between various locations. Systems are currently being developed to disinfect or otherwise sanitize areas within aircraft, for example, that use ultraviolet (UV) light.

During operation, UV light emitters typically consume power and generate heat. Accordingly, use of a persistent UV light emitter within an environment in which power and resources are limited may be impractical, as the persistent UV light emitter may draw more power than desired.

### SUMMARY OF THE DISCLOSURE

A need exists for a system and a method for effectively and efficiently operating UV light emitters. Further, a need exists for a system and a method for operating UV light emitters in environments in which resources are limited. Additionally, a need exists for a system and a method for allocating sanitizing resources within an environment.

With those needs in mind, certain embodiments of the present disclosure provide a system for sanitizing an enclosed space. The system includes one or more sensors within the enclosed space. The one or more sensors are configured to detect one or more actions within the enclosed space and output one or more action detection signals indicative of the one or more actions. One or more ultraviolet (UV) lamps are also within the enclosed space. The one or more UV lamps are configured to emit UV light in relation to one or more portions of the enclosed space. A sanitizing control unit is in communication with the one or more sensors and the one or more UV lamps. The sanitizing control unit is configured to receive the one or more action detection signals from the one or more sensors. The sanitizing control unit is also configured to modify operation of the one or more UV lamps in response to the one or more actions being a triggering event.

In at least one example, the enclosed space includes an internal cabin of a vehicle. As a further example, the one or more sensors are associated with one or more seats within the internal cabin.

In at least one example, the UV lamps are configured to emit the UV light within a far UV spectrum.

In at least one example, the triggering event includes one or both of a cough or a sneeze.

As an example, the one or more sensors comprise one or more of at least one microphone, at least one camera, at least one particulate sensor, or at least one pressure sensor. As another example, the one or more sensors include at least one microphone, at least one camera, at least one particulate sensor, and at least one pressure sensor.

In at least one example, the sanitizing control unit is configured to modify operation of the one or more UV lamps in response to the one or more actions being a triggering event by activating the one or more UV lamps to emit the UV light onto the one or more surfaces. In at least one other example, the sanitizing control unit is configured to modify operation of the one or more UV lamps in response to the one or more actions being a triggering event by increasing an intensity of the UV light.

In at least one example, the one or more sensors and the one or more UV lamps are integrated in a common housing.

In at least one example, the system also includes a resource allocation control unit in communication with the one or more sensors. The resource allocation control unit is configured to generate one or more resource recommendations regarding the one or more UV lamps for the enclosed space based on one or more of the triggering events.

Certain embodiments of the present disclosure provide a method for sanitizing an enclosed space. The method includes receiving, by a sanitizing control unit from one or more sensors within an enclosed space, one or more action detection signals indicative of one or more actions of one or more individuals within the enclosed space; and modifying, by the sanitizing control unit, operation of one or more ultraviolet (UV) lamps within the enclosed space in response to the one or more actions being a triggering event.

Certain embodiments of the present disclosure provide a system for allocating sanitizing resources within an enclosed space. The system includes one or more sensors within the enclosed space. The one or more sensors are configured to detect one or more actions within the enclosed space and output one or more action detection signals indicative of the one or more actions. A resource allocation control unit is in communication with the one or more sensors. The resource allocation control unit is configured to generate one or more resource recommendations regarding one or more ultraviolet (UV) lamps for the enclosed space based on one or more triggering events of the one or more actions.

In at least one example, the resource allocation control unit is configured to receive the one or more action detection signals from the one or more sensors. The resource allocation control unit is further configured to determine the one or more triggering events from the one or more action detection signals.

In at least one example, the resource allocation control unit stores data regarding the triggering events over a predetermined time in the memory. As a further example, the data includes locations of the triggering events. As a further example, the data also includes dates of the triggering events.

In at least one example, the resource allocation control unit is further configured to generate an event map that shows the areas of the triggering events over a predetermined period of time.

In at least one example, a user interface is in communication with the resource allocation control unit. The resource allocation control unit outputs a resource allocation signal including one or more resource recommendations to the user interface. The user interface shows the one or more resource recommendations on a display of the user interface.

Certain embodiments of the present disclosure provide a method for allocating sanitizing resources within an enclosed space. The method includes generating, by a resource allocation control unit, one or more resource recommendations regarding one or more ultraviolet (UV) lamps for the enclosed space based on one or more triggering events of one or more actions detected by one or more sensors within the enclosed space.

### BRIEF DESCRIPTION OF THE DRAWINGS

Figure 1 illustrates a schematic block diagram of a system for sanitizing one or more portions of an enclosed space.
Figure 2 illustrates a schematic block diagram of a system for sanitizing one or more portions of the enclosed space.
Figure 3 illustrates a flow chart of a method for sanitizing surfaces within the enclosed space.
Figure 4 illustrates a flow chart of a method for resource allocation within an enclosed space.
Figure 5 illustrates a schematic block diagram of a control unit.
Figure 6 illustrates a perspective front view of an aircraft.
Figure 7A illustrates a top plan view of an internal cabin of an aircraft.
Figure 7B illustrates a top plan view of an internal cabin of an aircraft.
Figure 8 illustrates a perspective interior view of an internal cabin of an aircraft.

### DETAILED DESCRIPTION OF THE DISCLOSURE

The foregoing summary, as well as the following detailed description of certain embodiments will be better understood when read in conjunction with the appended drawings. As used herein, an element or step recited in the singular and preceded by the word "a" or "an" should be understood as not necessarily excluding the plural of the elements or steps. Further, references to "one example" are not intended to be interpreted as excluding the existence of additional embodiments that also incorporate the recited features. Moreover, unless explicitly stated to the contrary, embodiments "comprising" or "having" an element or a plurality of elements having a particular condition can include additional elements not having that condition.

Certain embodiments of the present disclosure provide systems and methods for intelligent operation and allocation of a persistent ultraviolet (UV) light disinfection system. The systems and methods include one or more sensors and a detection algorithm that feeds into a control and power management system of one or more UV lamps. Examples of the sensors include microphones, cameras, particulate sensors, pressure sensors, and/or the like. For example, a microphone can be configured to detect a sound having a frequency associated within a cough or a sneeze. When such a sound is detected, the UV lamps can be activated and/or intensified to sanitize the area at and around where the sound was detected.

Certain embodiments of the present disclosure provide a sanitizing system that includes a plurality of ultraviolet (UV) lamps mounted at various locations within an enclosed space. The UV lamps are configured to receive electrical power from a power source and to emit UV light onto surfaces within the enclosed space. A control unit including one or more processors is operatively connected to the UV lamps. One or more sensors are disposed within the enclosed space and operatively connected to the control unit. The sensors are configured to detect actions (such as sounds or motions, for example, coughs and sneezes) originating from one or more individuals in the enclosed space. In at least one example, the control unit is configured to modify the electrical power supplied to one or more of the UV lamps located in the environment based on the detected event.

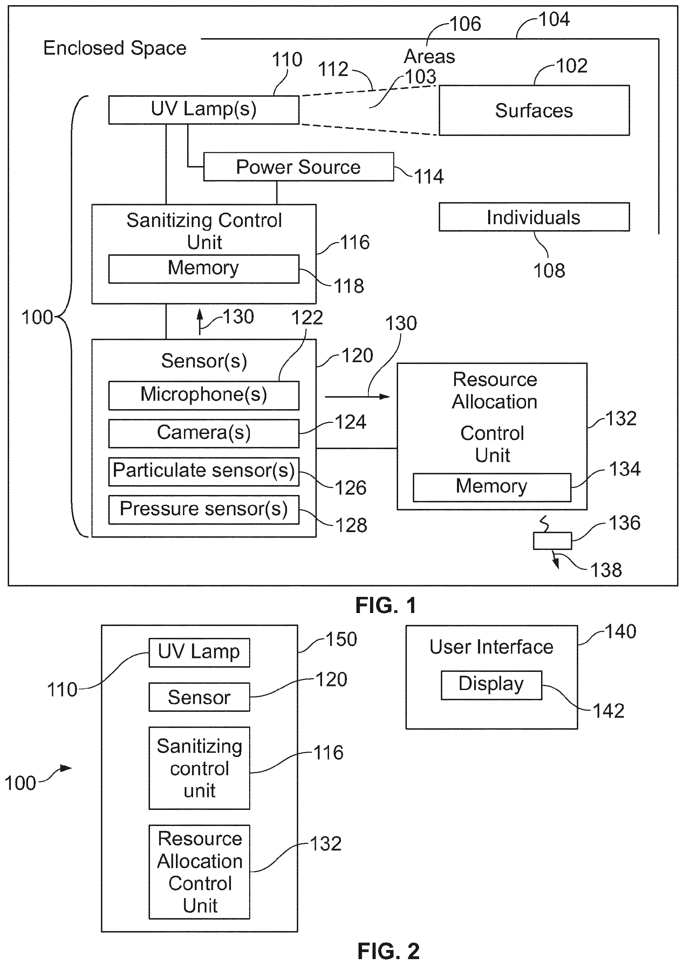

Figure 1 illustrates a schematic block diagram of a system 100 for sanitizing one or more portions of an enclosed space 104, according to an example of the present disclosure. The portions include one or more of surfaces 102, fluid 103 within (such as air, aerosols, etc.) an area 106, and/or the like. In at least one example, the enclosed space 104 is an internal cabin of a vehicle, such as a commercial aircraft. In at least one other example, the enclosed space 104 is one or more rooms within a residential or commercial building. As an example, the enclosed space 104 can be an operating room within a hospital. In at least one other example, the enclosed space 104 is an open air venue, such as a stadium, concert space, and/or the like.

The enclosed space 104 includes one or more areas 106. Individuals 108 are within the areas 106. For example, the individuals 108 are passengers within sections (examples of areas 106) within an internal cabin of a vehicle. The individuals 108 can be seated within the areas 106, such as within passenger seats of a commercial aircraft.

The surfaces 102 can be any physical surface within the enclosed space 104. For example, the surfaces 102 include walls, floors, ceilings, tables, countertops, seats, and/or the like within the enclosed space 104. In the example in which the enclosed space 104 is an internal cabin of a vehicle, the surfaces 102 includes floors, walls, ceilings, passenger seats, counters, drawers, cabinets, and/or the like, such as within a passenger cabin, galleys, lavatories, and/or the like.

One or more ultraviolet (UV) lamps 110 are disposed within the enclosed space 104. For example, a plurality of the UV lamps 110 are disposed throughout the enclosed space 104. The UV lamps 110 can be fixed structures that are mounted at various locations within the enclosed space 104. For example, the UV lamps 110 can be secured to walls, ceilings, floors, and/or the like within the enclosed space 104. As a further example, the UV lamps 110 secured to structure within the enclosed space 104 can be fixed in position, or optionally moveable (such as rotatable, pivotal, articulatable, and/or the like). As another example, the UV lamps 110 can be mobile systems that are moveable throughout the enclosed space 104. As an example, the UV lamps 110 can be within wand assemblies, coupled to a cart, part of a robotic sanitizing system, and/or the like.

The UV lamps 110 are configured to emit UV light 112 into the one or more portions, such as into the fluid 103, onto the surfaces 102, and/or the like. The UV light 112 sanitizes the surfaces 102, the fluid 103, and/or the like. In at least one example, the UV lamps 110 emit the UV light 112 in a far UV light spectrum, such as at a wavelength of 222 nm, which neutralizes (such as kills) microbes (for example, viruses and bacteria), while posing no risk to humans. The UV lamps 110 may be used within the enclosed space 104 to decontaminate and kill pathogens. Optionally, the UV lamps 110 can be configured to emit the UV light 112 at other wavelengths, such as the UVC spectrum (for example, at a wavelength of 254 nm).

The UV lamps 110 are coupled to a power source 114, which provides power to the UV lamps 110. For example, the power source 114 is a main power source, such as a source of alternating current power, within the enclosed space 104. As another example, the power source 114 can be one or more batteries. As another example, the power source 114 can be a distinct power source of the UV lamps 110. The UV lamps 110 are examples of sanitizing resources.

In at least one example, a UV lamp 110 can be associated with one or more seats within an internal cabin of a vehicle. For example, a UV lamp 110 can be mounted above one or more seats within the internal cabin, such as within one or more passenger service units (PSUs). As another example, a UV lamp 110 can be associated with a row of seats within the internal cabin. For example, a single UV lamp 110 can be disposed above a row of seats. As another example, a UV lamp 110 can be associated with a particular area within the internal cabin. For example, a UV lamp 110 can be mounted on a portion of a ceiling, wall, or the like within a galley, lavatory, section of seats within an internal cabin, and/or the like.

A sanitizing control unit 116 is in communication with the UV lamp 110 and/or the power source 114, such as through one or more wired or wireless connections. The sanitizing control unit 116 includes or is otherwise in communication with a memory 118, which stores data. In at least one example, the sanitizing control unit 116 is within the enclosed space 104. Optionally, the sanitizing control unit 116 can be remote from the enclosed space 104.

One or more sensors 120 are disposed within the enclosed space 104. The sensors 120 are in communication with the sanitizing control unit 116, such as through one or more wired or wireless connections. The sensors 120 are configured to detect actions within the enclosed space 104. In particular, the sensors 120 are configured to detect one or more actions of the individuals 108. The actions include sounds or motions. For example, the actions can be sounds associated with a sneeze or a cough. As another example, the actions include physical motions associated with a sneeze or a cough.

An example of a sensor 120 includes a microphone 122. The microphone 122 is configured to detect an action, such as a sound. In at least one example, the microphone 122 is configured to detect a sound associated with a cough or sneeze, such as a frequency of a sound that is correlated with a cough or a sneeze. Data associated with the sound is stored in the memory 118, for example.

An example of a sensor 120 includes a camera 124, such as a photographic camera, a video camera, an infrared camera, and/or the like. In at least one example, the camera 124 is configured to detect a visual indicator associated with a cough or sneeze, such as a plume of air and/or droplets exhaled by an individual 108, a physical lurch, head motion, facial motion, and/or the like that is correlated with a cough or a sneeze. Data associated with the visual indicator is stored in the memory 118, for example.

An example of a sensor 120 includes a particulate sensor 126, such as a chemical sensor that is configured to detect particulate matter within the air. In at least one example, the particulate sensor 126 is configured to detect particulates associated with a cough or sneeze that are expelled by the individuals 108. Data associated with the particulate matter is stored in the memory 118, for example.

An example of a sensor 120 includes a pressure sensor, which is configured to detect a pressure change associated with a cough or sneeze. Data associated with the pressure change is stored in the memory 118, for example.

An example of a sensor 120 includes a chemical sensor, which is configured to detect one or more chemicals. The chemicals may be detected on a surface, within an aerosol, air, or other such fluid, and/or the like.

The sensors 120 can be dedicated sensors that are fixed in position within the enclosed space 104. As another example, the sensors 120 can be moveable within the enclosed space 104. In at least one example, the sensors 120 can be part of handheld devices, such as smart phones or tablets, of individuals, attendants, or the like within the enclosed space 104.

In at least one example, the sensors 120 includes an array of the sensors 120 (such as microphones 122, cameras 124, particulate sensors 126, and/or pressure sensors 128) distributed within an environment, such as the enclosed space 104. In at least one example, a sensor 120 can be associated with one or more seats within an internal cabin of a vehicle. For example, a sensor 120 can be mounted above one or more seats within the internal cabin, such as within one or more passenger service units (PSUs). As another example, a sensor 120 can be mounted on or within a seat within the internal cabin. As another example, a sensor 120 can be associated with a row of seats within the internal cabin. For example, a single sensor 120 can be disposed above a row of seats. As another example, a sensor 120 can be associated with a particular area within the internal cabin. For example, a sensor 120 can be mounted on a portion of a ceiling, wall, or the like within a galley, lavatory, section of seats within an internal cabin, and/or the like.

Figure 1 shows examples of sensors 120. The sensors 120 can include more or less than the examples shown. For example, in at least one example, the sensors 120 include only acoustic sensors, such as the microphones 122. As another example, the sensors 120 can include only the cameras 124. As another example, the sensors 120 can include at least two of the microphones 122, the cameras 124, the particulate sensors 126, and/or the pressure sensors 128.

In operation, the sanitizing control unit 116 operates the UV lamps 110 to sanitize the surfaces 102 in response to the sensors 120 detecting one or more actions of the individuals 108. In at least one example, the sanitizing control unit 116 activates the one or more UV lamps 110 in response to the one or more sensors 120 detecting that the detected action of the individual 108 is a triggering event. For example, the action is at a frequency or decibel level that is associated with a sneeze or a cough. As another example, the action includes a visual indication, such as an exhaled plume of fluid, that is associated with a sneeze or a cough. One or more of the sensors 120 detects the action originating from the individual 108 within the particular area 106 of the enclosed space. For example, the microphones 122, the cameras 124, the particulate sensors 126, and/or the pressure sensors 128 detect the action and the location thereof. The sensor(s) 120 output one or more action detection signals 130 indicative of the action to the sanitizing control unit 116. The sanitizing control unit 116 analyzes the action detection signals 130 with the data stored in the memory 118 to determine if the action is a triggering event (for example, is at a frequency or decibel level associated with a sneeze or a cough, includes a visual indication associated with a sneeze or a cough, or the like). The triggering event is such that it triggers the sanitizing control unit 116 to change an operational status of the UV lamp(s) 110 at and/or proximate (such as within 20 feet or less) the location of the action. For example, the data stored in the memory 118 includes data regarding the frequency, physical motion, particulate content, pressure, and/or the like associated with a sneeze, cough, or other such triggering event.

If the sanitizing control unit 116 determines that the action of the individual is a triggering event (such as a cough or sneeze), the sanitizing control unit 116 alters operation of the UV lamp 110 at and/or proximate the location of the triggering event. For example, the sanitizing control unit 116 can activate the UV lamp 110 to emit the UV light 112 onto one or more surfaces 102 proximate to the triggering event (such as within a radius of 10 feet or less) to sanitize one or more surfaces 102 at and/or surrounding the triggering event. The sanitizing control unit 116 can operate the UV lamp(s) 110 in an activated state for a predetermined sanitizing period, such as 5 seconds or less, then deactivate the UV lamp(s) 110. As another example, the UV lamp(s) 110 can be persistent UV lamps 110 that emit the UV light 112 at a first intensity. In response to the triggering event, the sanitizing control unit 116 can modify power (for example, increase the power) from the power source 114 to the UV lamp(s) 110 so that the UV lamp(s) 110 emit the UV light 112 at a second intensity, which is greater than the first intensity, for a predetermined increased intensity period, such as 5 seconds or less. As such, in response to the triggering event (such as a sneeze or cough, as detected by the sensor(s) 120), the sanitizing control unit 116 alters operation of the UV lamp(s) 110, such as by activating the UV lamp(s) 110, or increasing the intensity of the emitted UV light 112, for a predetermined period, such as 5 seconds or less. Optionally, the predetermined period can be more than 5 seconds. For example, the predetermined period can be 10 seconds, 20 seconds, 30 seconds, or more.

In at least one example, the UV lamps 110 are part of a persistent UV sanitizing system. The sanitizing control unit 116 controls operation of the UV lamps 110 based on detection of triggering events of the individuals 108, as detected by the sensors 120. The system 100 provides a safer and more efficient sanitization by modifying operation of the UV lamps 110 (such as through controlling power thereto) based on the detection the triggering event, such as a cough or sneeze.

In at least one example, the sanitizing control unit 116 analyzes the action detection signals 130 output by the sensors 120 in relation to a complex neural network stored in the memory 118. The complex neural network is trained to detect actions such as coughs and sneezes, or other deep learning techniques that detect patterns based on activation functions and feedback loops. The action detection signals 130 include the location of an action (such as a cough, sneeze, or the like). If the action detection signal 130 is a triggering event, such as a cough or sneeze, the sanitizing control unit 116 modifies operation of the UV lamp(s) 110 accordingly. For example, the sanitizing control unit 116 determines the location in the enclosed space 104 where the triggering event occurred, and activates different patterns of lights, varies the power and intensity of the UV lamp(s) 110 in relation to the triggering event, and/or the like.

In at least one example, the sanitizing control unit 116 determines a center point of the triggering event, and controls irradiance of the UV lamp(s) 110 so that a higher dose of UV light 112 is focused at the center point, and a lower dose is further away (such as 10 feet away) from the center point. In at least one example, when the enclosed space 104 is an internal cabin of a commercial aircraft, the power to the UV lamps 110 can be further controlled by phase of flight, or can also respond to passenger movement or any other type of input, either from a sensor network, or manual intervention by flight crew.

As shown, the UV lamps 110 can be separate and distinct from the sensors 120 and the sanitizing control unit 116. Optionally, the UV lamps 110 can be integrated with the sensors 120 and/or the sainting control unit 116. For example, one or more of the UV lamps 110 can further include one or more of the sensors 120 and/or the sanitizing control unit 116.

By modifying operation of the UV lamps 110 in response to a triggering event (such as a cough or sneeze) as detected by the sensors 120 (in contrast to constantly emitting UV light from the UV lamps 110 at the same intensity), the system 100 reduces overall power consumption, lowers cooling requirements, generates significantly less ozone, and does not require the oversight of persons to track all cough and sneeze sources throughout the enclosed space 104. The system 100 is also adaptable to different contagions by allowing the potential for automated changes in how long the UV lamps 110 remain active, as well as new area configurations.

As described herein, in at least one example, the system 100 for sanitizing the enclosed space 104 includes the one or more sensors 120 within the enclosed space 104. The one or more sensors 120 are configured to detect one or more actions within the enclosed space 104 and output one or more action detection signals 130 indicative of the one or more actions. One or more ultraviolet (UV) lamps 110 are also within the enclosed space 104. The one or more UV lamps 110 are configured to emit UV light 112 onto one or more surfaces 102 within the enclosed space 104. The sanitizing control unit 116 is in communication with the one or more sensors 120 and the one or more UV lamps 110. The sanitizing control unit 116 is configured to receive the one or more action detection signals 130 from the one or more sensors 120. The sanitizing control unit 116 is further configured to modify operation of the one or more UV lamps 110 in response to the one or more actions being a triggering event (such as a cough, sneeze, yelling (for example, audio signals above a predetermined decibel level), and/or the like).

In at least one example, a resource allocation control unit 132 is in communication with the sensors 120 through one or more wired or wireless connections. The resource allocation control unit 132 can be separate and distinct from the sanitizing control unit 116. Optionally, the resource allocation control unit 132 and the sanitizing control unit 116 can be part of a common control unit, such as a within a common integrated chip, for example.

In operation, the resource allocation control unit 132 monitors the action detection signals 130 output from the sensors 120. The resource allocation control unit 132 analyzes the action detection signals 130 to determine the locations of the actions as detected by the sensors 120. The resource allocation control unit 132 is coupled to or otherwise includes a memory 134, which stores data regarding the various areas 106 of the enclosed space 104.

In response to receiving the action detection signals 130, the resource allocation control unit 132 determines if the actions as indicated from the action detection signals 130 are triggering events, as described above. In at least one example, the resource allocation control unit 132 is in communication with the sanitizing control unit 116, such as through one or more wired or wireless connections. The sanitizing control unit 116 can determine if the actions are triggering events, instead of the resource allocation control unit 132 separately determining whether the actions are triggering events.

The resource allocation control unit 132 stores data regarding the triggering events in the memory 134. The data regarding the triggering events includes the locations of the triggering events, and can also include dates and times of the triggering events. The resource allocation control unit 132 complies the data regarding the triggering events over a predetermined period of time. The predetermined period of time can be a single trip (such as a flight), multiple trips (such as 5 or more trips), a day, multiple days, a month, multiple months, a year, and/or the like. The greater the predetermined time, the more robust the data.

The resource allocation control unit 132 may then generate an event map 136, which shows the areas of the triggering events over the predetermined period of time. For example, the event map 136 shows frequency of triggering events at various locations within the enclosed space. The event map 136 shows areas where triggering events occur more frequently. Similarly, the event map 136 shows areas where the triggering events occur less frequently. Accordingly, the event map 136 can be used to determine where to locate sanitizing resources, such as the UV lamps 110 within the enclosed space 104.

In at least one example, the resource allocation control unit 132 provides resource recommendations for the enclosed space 104. The resource recommendations include the number of sanitizing resources (such as the UV lamps 110) and locations of the sanitizing resources within the enclosed space 104. For example, the resource recommendations indicate areas for the UV lamps 110 based on thresholds stored in the memory 134. For example, if the triggering events occur in an area 106 at least a certain predetermined number of times (such as 5, 10, 20, or more) over the predetermined period of time, the resource allocation control unit 132 recommends one or more UV lamps 110 to be located in that area. Conversely, if the triggering events occur in the areas 106 less than a certain predetermined number of times over the predetermined period of time, the resource allocation control unit 132 recommends that one or more UV lamps 110 not be located in that area. In this manner, the resource allocation control unit 132 is configured to determine efficient and effective location of the UV lamps 110 within the enclosed area 106. The UV lamps 110 can be shifted and changed based on the analysis performed by the resource allocation control unit 132.

The resource allocation control unit 132 outputs a resource allocation signal 138 that includes data regarding the determined areas of the triggering events, the event map 136, and/or resource recommendations. The resource allocation signal 138 can be received by a user interface 140, such as may be in communication with the resource allocation control unit 132 through one or more wired or wireless connections. The user interface 140 can be within the enclosed space 104 or remote therefrom. The user interface 140 can be a computer workstation, a laptop computer, a handheld smart device, and/or the like. The user interface 140 includes an electronic display 142, such as a monitor, television, touchscreen interface, and/or the like. Information regarding the resource allocation signal 138 can be presented on the display 142.

In at least one example, the resource allocation control unit 132 analyzes the action detection signals 130 to determine the location of the triggering events and generate the event map 136 for the enclosed space 104, and/or another similar enclosed space 104. In this manner, the resource allocation control unit 132 can be used to determine locations for sanitizing resources, such as UV lamps 110, for a future enclosed space (for example, an internal cabin of a commercial aircraft that is being manufactured, or is to be manufactured). As such, the resource allocation control unit 132 can be used in a system that may or may not include the UV lamps 110 and/or the sanitizing control unit 116. In at least one other example, the system 100 may not include the resource allocation control unit 132.

As described herein, in at least one example, the system 100 for allocating sanitizing resources within the enclosed space 104 includes one or more sensors 120 within the enclosed space 104. The one or more sensors 120 are configured to detect one or more actions within the enclosed space and output one or more action detection signals 130 indicative of the one or more actions. A resource allocation control unit 132 is in communication with the one or more sensors 120. The resource allocation control unit 132 is configured to receive the one or more action detection signals 130 from the one or more sensors 120. The resource allocation control unit 132 is further configured to generate one or more resource recommendations regarding one or more ultraviolet (UV) lamps 110 for the enclosed space 104 based on one or more triggering events of the one or more actions. In at least one example, the UV lamps 110 can be positioned within the enclosed space 104 based on the resource recommendation(s).

In at least one example, the UV lamp 110 can include one or more of the sensors 120. Further, the sanitation control unit 116 can be housed within the UV lamp 110. Additionally, the resource allocation control unit 132 can be housed within the UV lamp 110. As shown, the sanitation control unit 116 and the resource allocation control unit 132 can be separate and distinct control units. Optionally, the sanitation control unit 116 and the resource allocation control unit 132 can be part of an integrated processing system, such as within an integrated chip.

Figure 2 illustrates a schematic block diagram of the system 100 for sanitizing surfaces within the enclosed space, according to an example of the present disclosure. The system 100 can include one or more components integrated into a common housing 150. For example, the housing 150 includes a UV lamp 110, a sensor 120, the sanitizing control unit 116, and the resource control unit 132.

In at least one other example, the housing 150 does not include the resource allocation control unit 132. Instead, the resource allocation control unit 132 can be separate and distinct from the housing 150.

In at least one other example, the housing 150 does not include the sanitizing control unit 116. Instead, the sanitizing control unit 116 can be separate and distinct from the housing 150.

In at least one example, the housing 150 includes the UV lamp 110 and the sensor 120, but not the sanitizing control unit 116 or the resource allocation control unit 132. Referring again to Figure 1, in at least one example, the UV lamps 110, the sensors 120, the sanitizing control unit 116, and the resource allocation control unit 132 can all be separate and distinct from one another (that is, not integrated into a common housing).

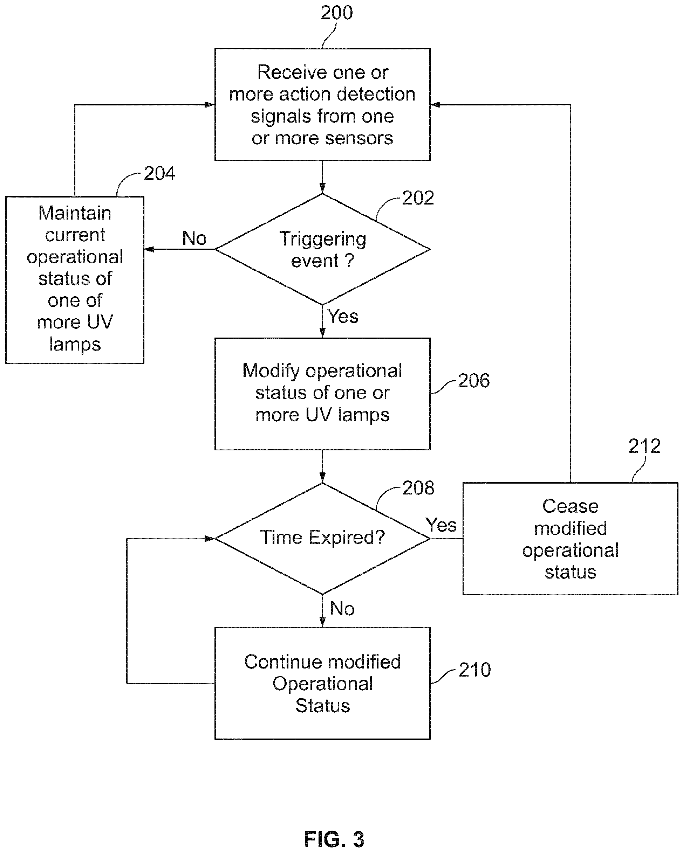

Figure 3 illustrates a flow chart of a method for sanitizing surfaces within the enclosed space, according to an example of the present disclosure. Referring to Figures 1 and 3, the method begins at 200, at which the sanitizing control unit 116 receives one or more action detection signals 130 from the one or more sensors 120. At 202, the sanitizing control unit 116 determines if the action detection signals 130 are indicative of a triggering event. If not, the method proceeds to 204, at which the sanitizing control unit 116 maintains the current operational status (for example, deactivated, or active at a first intensity) of the one or more UV lamps 110.

If, however, the sanitizing control unit 116 determines that the action detection signal 130 is indicative of a triggering event at 202, the method proceeds to 206, at which the sanitizing control unit 116 modifies the operational status (for example, activates, or operates at a second intensity that is greater than the first intensity) of the one or more UV lamps 110.

At 208, the sanitizing control unit 116 determines if the time for modified operational status has expired. For example, the time for the modified operational status is a time during which it is determined that the modified operation will effectively sanitize the surface(s) 102 proximate to the triggering event (such as 3-5 seconds of higher intensity UV light). If the time has not expired, the method proceeds to 210, at which the sanitizing control unit 116 continues the modified operational status of the UV lamp(s) 110. The method then returns to 208.

If, at 208, the time has expired, the method proceeds to 212, at which the sanitizing control unit 116 ceases the modified operational status of the UV lamp(s) 110. The method then returns to 200.

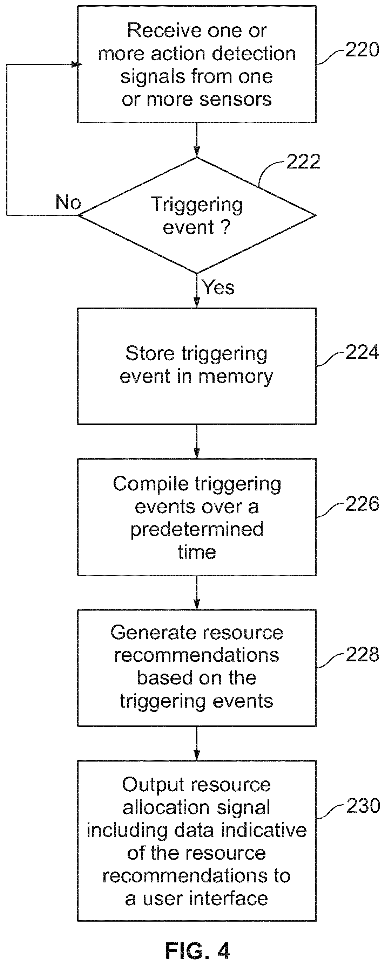

Figure 4 illustrates a flow chart of a method for resource allocation within an enclosed space, according to an example of the present disclosure. Referring to Figures 1 and 4, the begins at 220, at which the resource allocation control unit 132 receives one or more action detection signals 130 from the one or more sensors 120. At 222, the resource allocation control unit 222 determines if the action detection signals 130 are indicative of triggering events. If not, the process the returns to 220.

If, however, one or more of the action detection signals 130 is indicative of a triggering event, the resource allocation control unit 132 stores the triggering event in the memory 134. At 226, the resource allocation control unit 132 complies the triggering events over a predetermined time (such as a flight or other such trip, multiple flights/trips, a day, multiple days, a month, multiple months, and/or a year or more). At 228, the resource allocation control unit 132 generates resource recommendations for an enclosed space 104 based on the triggering events stored in the memory 134. At 230, the resource allocation control unit 132 outputs a resource allocation signal 138 including data indicative of the resource recommendations to the user interface 140.

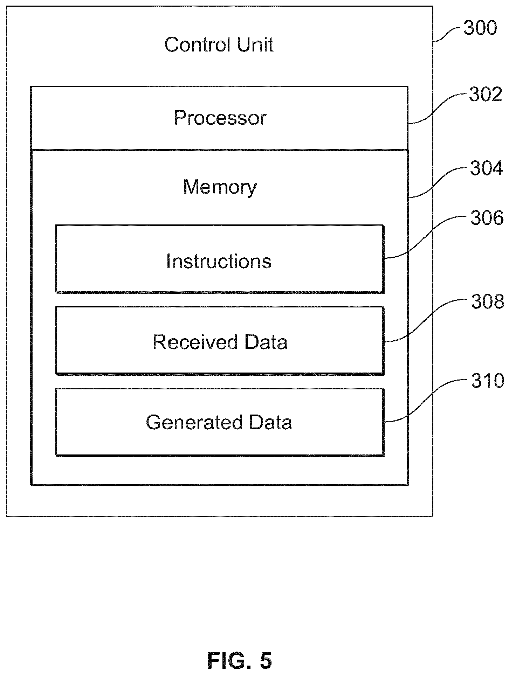

Figure 5 illustrates a schematic block diagram of a control unit 300, according to an example of the subject disclosure. The sanitizing control unit 116 and the resource allocation control unit 132 shown in Figure 1 can be configured as shown in Figure 5. In at least one example, the control unit 300 includes at least one processor 302 in communication with a memory 304. The memory 304 stores instructions 306, received data 308, and generated data 310. The control unit 300 shown in Figure 5 is merely exemplary, and non-limiting.

As used herein, the term "control unit," "central processing unit," "unit," "CPU," "computer," or the like can include any processor-based or microprocessor-based system including systems using microcontrollers, reduced instruction set computers (RISC), application specific integrated circuits (ASICs), logic circuits, and any other circuit or processor including hardware, software, or a combination thereof capable of executing the functions described herein. Such are exemplary only, and are thus not intended to limit in any way the definition and/or meaning of such terms. For example, the sanitizing control unit 116 and the resource allocation control unit 132 can be or include one or more processors that are configured to control operation thereof, as described herein.

The control unit(s), such as the sanitizing control unit 116 and the resource allocation control unit 132, are configured to execute a set of instructions that are stored in one or more data storage units or elements (such as one or more memories), in order to process data. For example, the sanitizing control unit 116 and the resource allocation control unit 132 can include or be coupled to one or more memories. The data storage units can also store data or other information as desired or needed. The data storage units can be in the form of an information source or a physical memory element within a processing machine. The one or more data storage units or elements can comprise volatile memory or nonvolatile memory, or can include both volatile and nonvolatile memory. As an example, the nonvolatile memory can comprise read only memory (ROM), programmable ROM (PROM), electrically programmable ROM (EPROM), electrically erasable PROM (EEPROM), and/or flash memory and volatile memory can include random access memory (RAM), which can act as external cache memory. The data stores of the disclosed systems and methods is intended to comprise, without being limited to, these and any other suitable types of memory.

The set of instructions can include various commands that instruct the control unit(s), such as the sanitizing control unit 116 and the resource allocation control unit 132, as a processing machine to perform specific operations such as the methods and processes of the various embodiments of the subject matter described herein. The set of instructions can be in the form of a software program. The software can be in various forms such as system software or application software. Further, the software can be in the form of a collection of separate programs, a program subset within a larger program or a portion of a program. The software can also include modular programming in the form of object-oriented programming. The processing of input data by the processing machine can be in response to user commands, or in response to results of previous processing, or in response to a request made by another processing machine.

The diagrams of embodiments herein can illustrate one or more control or processing units, such as the sanitizing control unit 116 and the resource allocation control unit 132. It is to be understood that the processing or control units can represent circuits, circuitry, or portions thereof that can be implemented as hardware with associated instructions (e.g., software stored on a tangible and non-transitory computer readable storage medium, such as a computer hard drive, ROM, RAM, or the like) that perform the operations described herein. The hardware can include state machine circuitry hardwired to perform the functions described herein. Optionally, the hardware can include electronic circuits that include and/or are connected to one or more logic-based devices, such as microprocessors, processors, controllers, or the like. Optionally, the control unit(s), such as the sanitizing control unit 116 and the resource allocation control unit 132, can represent processing circuitry such as one or more of a field programmable gate array (FPGA), application specific integrated circuit (ASIC), microprocessor(s), and/or the like. The circuits in various embodiments can be configured to execute one or more algorithms to perform functions described herein. The one or more algorithms can include aspects of embodiments disclosed herein, whether or not expressly identified in a flowchart or a method.

As used herein, the terms "software" and "firmware" are interchangeable, and include any computer program stored in a data storage unit (for example, one or more memories) for execution by a computer, including RAM memory, ROM memory, EPROM memory, EEPROM memory, and non-volatile RAM (NVRAM) memory. The above data storage unit types are exemplary only, and are thus not limiting as to the types of memory usable for storage of a computer program.

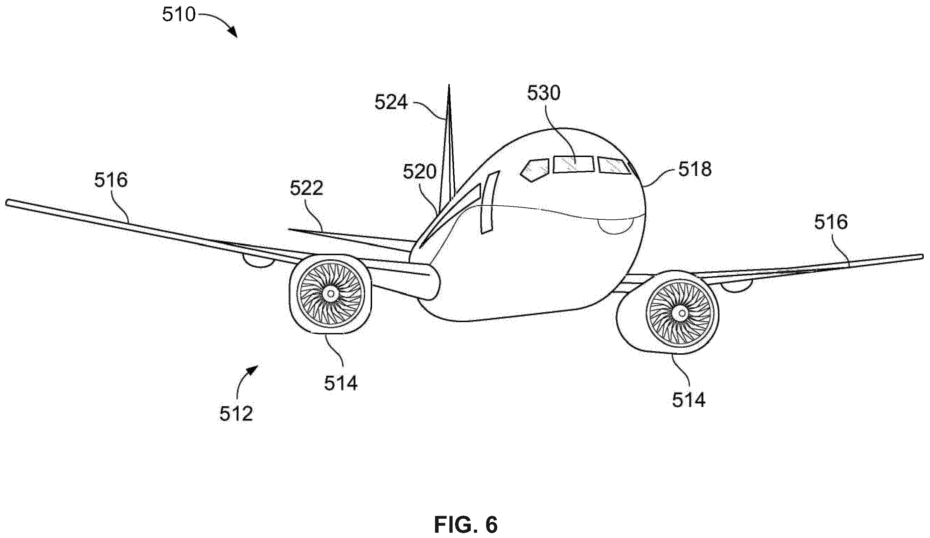

Figure 6 illustrates a perspective front view of an aircraft 510, according to an example of the present disclosure. The aircraft 510 includes a propulsion system 512 that includes engines 514, for example. Optionally, the propulsion system 512 may include more engines 514 than shown. The engines 514 are carried by wings 516 of the aircraft 510. In other embodiments, the engines 514 may be carried by a fuselage 518 and/or an empennage 520. The empennage 520 may also support horizontal stabilizers 522 and a vertical stabilizer 524.

The fuselage 518 of the aircraft 510 defines an internal cabin 530, which includes a flight deck or cockpit, one or more work sections (for example, galleys, personnel carry-on baggage areas, and the like), one or more passenger sections (for example, first class, business class, and coach sections), one or more lavatories, and/or the like. The internal cabin 530 is an example of an enclosed space 104, as shown in Figure 1.

Alternatively, instead of an aircraft, embodiments of the present disclosure may be used with various other vehicles, such as automobiles, buses, locomotives and train cars, watercraft, and the like. Further, embodiments of the present disclosure may be used with respect to fixed structures, such as commercial and residential buildings. In general, the sanitizing systems described herein may be used to sanitizing various components, such as within enclosed spaces, outdoor spaces, and the like.

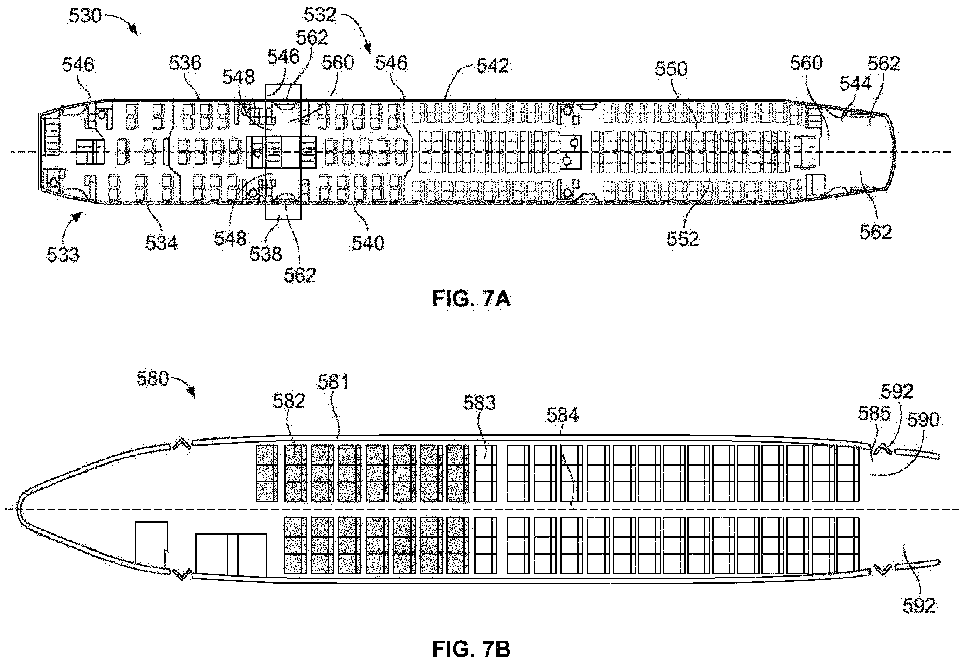

Figure 7A illustrates a top plan view of an internal cabin 530 of an aircraft, according to an example of the present disclosure. The internal cabin 530 may be within the fuselage 532 of the aircraft, such as the fuselage 518 of Figure 6. For example, one or more fuselage walls may define the internal cabin 530. The internal cabin 530 includes multiple areas, including a front section 533, a first-class section 534, a business class section 536, a front galley station 538, an expanded economy or coach section 540, a standard economy of coach section 542, and an aft section 544, which may include multiple lavatories and galley stations. It is to be understood that the internal cabin 530 may include more or less areas than shown. For example, the internal cabin 530 may not include a first-class section, and may include more or less galley stations than shown. Each of the sections may be separated by a cabin transition area 546, which may include class divider assemblies between aisles 548.

As shown in Figure 7A, the internal cabin 530 includes two aisles 550 and 552 that lead to the aft section 544. Optionally, the internal cabin 530 may have less or more aisles than shown. For example, the internal cabin 530 may include a single aisle that extends through the center of the internal cabin 530 that leads to the aft section 544.

The aisles 548, 550, and 552 extend to egress paths or door passageways 560. Exit doors 562 are located at ends of the egress paths 560. The egress paths 560 may be perpendicular to the aisles 548, 550, and 552. The internal cabin 530 may include more egress paths 560 at different locations than shown. The sanitizing systems shown and described with respect to Figures 1-5 may be used to sanitize various surfaces within the internal cabin 530, such as passenger seats, monuments, stowage bin assemblies, components on and within lavatories, galley equipment and components, and/or the like.

Figure 7B illustrates a top plan view of an internal cabin 580 of an aircraft, according to an example of the present disclosure. The internal cabin 580 is an example of the internal cabin 530 shown in Figure 6. The internal cabin 580 may be within a fuselage 581 of the aircraft. For example, one or more fuselage walls may define the internal cabin 580. The internal cabin 580 includes multiple areas, including a main cabin 582 having passenger seats 583, and an aft section 585 behind the main cabin 582. It is to be understood that the internal cabin 580 may include more or less areas than shown.

The internal cabin 580 may include a single aisle 584 that leads to the aft section 585. The single aisle 584 may extend through the center of the internal cabin 580 that leads to the aft section 585. For example, the single aisle 584 may be coaxially aligned with a central longitudinal plane of the internal cabin 580.

The aisle 584 extends to an egress path or door passageway 590. Exit doors 592 are located at ends of the egress path 590. The egress path 590 may be perpendicular to the aisle 584. The internal cabin 580 may include more egress paths than shown. The sanitizing systems shown and described with respect to Figures 1-5 may be used to sanitize various surfaces within the internal cabin 530, such as passenger seats, monuments, stowage bin assemblies, components on and within lavatories, galley equipment and components, and/or the like.

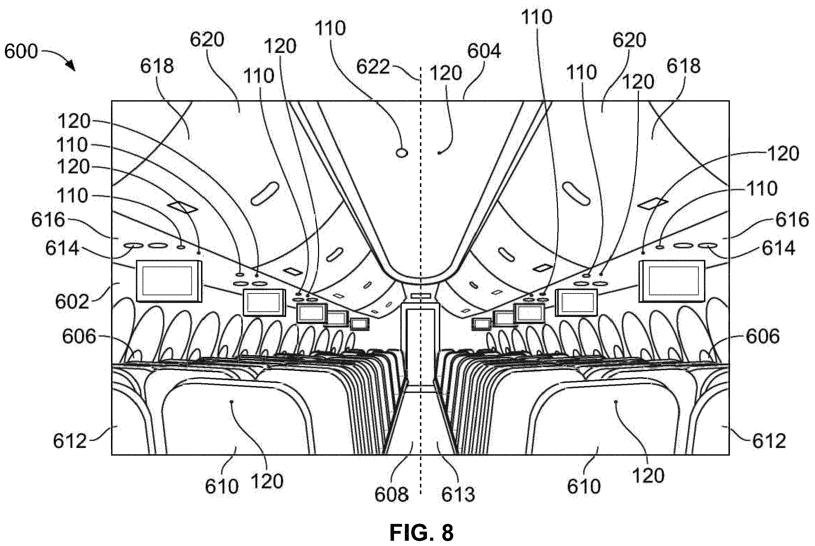

Figure 8 illustrates a perspective interior view of an internal cabin 600 of an aircraft, according to an example of the present disclosure. The internal cabin 600 includes outboard walls 602 connected to a ceiling 604. Windows 606 may be formed within the outboard walls 602. A floor 608 supports rows of seats 610. As shown in Figure 8, a row 612 may include two seats 610 on either side of an aisle 613. However, the row 612 may include more or less seats 610 than shown. Additionally, the internal cabin 600 may include more aisles than shown.

Passenger service units (PSUs) 614 are secured between an outboard wall 602 and the ceiling 604 on either side of the aisle 613. The PSUs 614 extend between a front end and rear end of the internal cabin 600. For example, a PSU 614 may be positioned over each seat 610 within a row 612. Each PSU 614 may include a housing 616 that generally contains vents, reading lights, an oxygen bag drop panel, an attendant request button, and other such controls over each seat 610 (or groups of seats) within a row 612.

Overhead stowage bin assemblies 618 are secured to the ceiling 604 and/or the outboard wall 602 above and inboard from the PSU 614 on either side of the aisle 613. The overhead stowage bin assemblies 618 are secured over the seats 610. The overhead stowage bin assemblies 618 extend between the front and rear end of the internal cabin 600. Each stowage bin assembly 618 may include a pivot bin or bucket 620 pivotally secured to a strongback. The overhead stowage bin assemblies 618 may be positioned above and inboard from lower surfaces of the PSUs 614. The overhead stowage bin assemblies 618 are configured to be pivoted open in order to receive passenger carry-on baggage and personal items, for example.

As used herein, the term "outboard" means a position that is further away from a central longitudinal plane 622 of the internal cabin 600 as compared to another component. The term "inboard" means a position that is closer to the central longitudinal plane 622 of the internal cabin 600 as compared to another component. For example, a lower surface of a PSU 614 may be outboard in relation to a stowage bin assembly 618.

The sanitizing systems shown and described with respect to Figures 1-5 may be used to sanitize various structures shown within the internal cabin 600. As shown, numerous UV lamps 110 and sensors 120 can be disposed throughout the internal cabin 600. As examples, UV lamps 110 can be secured to PSUs 614, the ceiling 604, walls 602, and/or the like. Further, the sensors 120 can be secured to PSUs 614, the ceiling 604, walls 602, seats 610, and/or the like.

Referring to Figures 1-7, embodiments of the subject disclosure provide systems and methods that allow large amounts of data to be quickly and efficiently analyzed by a computing device. For example, the behavior of numerous individuals 108 within an enclosed space 104 (such as an internal cabin of an aircraft) are monitored, as described herein. As such, large amounts of data are being tracked and analyzed. The vast amounts of data are efficiently organized and/or analyzed by the sanitizing control unit 116 and/or the resource allocation control unit 132, as described herein. The sanitizing control unit 116 and/or the resource allocation control unit 132 analyzes the data in a relatively short time in order to quickly and efficiently determine where and when to modify the UV lamps 110, determined recommendations for resource allocation, and/or the like for the enclosed space 104. A human being would be incapable of efficiently analyzing such vast amounts of data in such a short time. As such, embodiments of the subject disclosure provide increased and efficient functionality, and vastly superior performance in relation to a human being analyzing the vast amounts of data.

In at least one example, components of the system 100, such as the sanitizing control unit 116 and/or the resource allocation control unit 132, provide and/or enable a computer system to operate as a special computer system for sanitizing surfaces within an enclosed space, generating resource allocation recommendations, and/or the like for enclosed space 104.

Further, the disclosure comprises embodiments according to the following clauses:
Clause 1. A system for sanitizing an enclosed space, the system comprising:
   one or more sensors within the enclosed space, wherein the one or more sensors are configured to detect one or more actions within the enclosed space and output one or more action detection signals indicative of the one or more actions;
   one or more ultraviolet (UV) lamps within the enclosed space, wherein the one or more UV lamps are configured to emit UV light in relation to one or more portions of the enclosed space; and
   a sanitizing control unit in communication with the one or more sensors and the one or more UV lamps, wherein the sanitizing control unit is configured to receive the one or more action detection signals from the one or more sensors, and wherein the sanitizing control unit is configured to modify operation of the one or more UV lamps in response to the one or more actions being a triggering event.
Clause 2. The system of Clause 1, wherein the enclosed space comprises an internal cabin of a vehicle.
Clause 3. The system of Clause 2, wherein the one or more sensors are associated with one or more seats within the internal cabin.
Clause 4. The system of any of Clauses 1-3, wherein the UV lamps are configured to emit the UV light within a far UV spectrum.
Clause 5. The system of any of Clauses 1-4, wherein the triggering event comprises one or both of a cough or a sneeze.
Clause 6. The system of any of Clauses 1-5, wherein the one or more sensors comprise one or more of at least one microphone, at least one camera, at least one particulate sensor, or at least one pressure sensor.
Clause 7. The system of any of Clauses 1-6, wherein the one or more sensors comprises at least one microphone, at least one camera, at least one particulate sensor, and at least one pressure sensor.
Clause 8. The system of any of Clauses 1-7, wherein the sanitizing control unit is configured to modify operation of the one or more UV lamps in response to the one or more actions being a triggering event by activating the one or more UV lamps to emit the UV light onto the one or more surfaces.
Clause 9. The system of any of Clauses 1-8, wherein the sanitizing control unit is configured to modify operation of the one or more UV lamps in response to the one or more actions being a triggering event by increasing an intensity of the UV light.
Clause 10. The system of any of Clauses 1-9, wherein the one or more sensors and the one or more UV lamps are integrated in a common housing.
Clause 11. The system of any of Clauses 1-10, further comprising a resource allocation control unit in communication with the one or more sensors, wherein the resource allocation control unit is configured to generate one or more resource recommendations regarding the one or more UV lamps for the enclosed space based on one or more of the triggering events.
Clause 12. A method for sanitizing an enclosed space, the method comprising:
   receiving, by a sanitizing control unit from one or more sensors within an enclosed space, one or more action detection signals indicative of one or more actions of one or more individuals within the enclosed space; and
   modifying, by the sanitizing control unit, operation of one or more ultraviolet (UV) lamps within the enclosed space in response to the one or more actions being a triggering event.
Clause 13. The method of Clause 12, wherein the enclosed space comprises an internal cabin of a vehicle.
Clause 14. The method of Clause 13, wherein the one or more sensors are associated with one or more seats within the internal cabin.
Clause 15. The method of any of Clauses 12-14, wherein the triggering event comprises one or both of a cough or a sneeze.
Clause 16. The method of any of Clauses 12-15, wherein the one or more sensors comprise one or more of at least one microphone, at least one camera, at least one particulate sensor, or at least one pressure sensor.
Clause 17. The method of any of Clauses 12-16, wherein said modifying comprises activating the one or more UV lamps to emit UV light in relation to one or more portions of the enclosed space.
Clause 18. The method of any of Clauses 12-17, wherein said modifying increasing an intensity of UV light emitted from the one or more UV lamps.
Clause 19. The method of any of Clauses 12-18, further comprising integrating the one or more sensors and the one or more UV lamps in a common housing.
Clause 20. The method of any of Clauses 12-19, further comprising generating, by a resource allocation control unit in communication with the one or more sensors, one or more resource recommendations regarding the one or more UV lamps for the enclosed space based on one or more of the triggering events.
Clause 21. A system for allocating sanitizing resources within an enclosed space, the system comprising:
   one or more sensors within the enclosed space, wherein the one or more sensors are configured to detect one or more actions within the enclosed space and output one or more action detection signals indicative of the one or more actions; and
   a resource allocation control unit in communication with the one or more sensors, wherein the resource allocation control unit is configured to generate one or more resource recommendations regarding one or more ultraviolet (UV) lamps for the enclosed space based on one or more triggering events of the one or more actions.
Clause 22. The system of Clause 21, wherein the resource allocation control unit is configured to receive the one or more action detection signals from the one or more sensors, and wherein the resource allocation control unit is configured to determine the one or more triggering events from the one or more action detection signals.
Clause 23. The system of Clauses 21 or 22, wherein the resource allocation control unit stores data regarding the triggering events over a predetermined time in the memory.
Clause 24. The system of Clause 23, wherein the data comprises locations of the triggering events.
Clause 25. The system of Clause 24, wherein the data further comprises dates of the triggering events.
Clause 26. The system of any of Clauses 21-25, wherein the resource allocation control unit is further configured to generate an event map that shows the areas of the triggering events over a predetermined period of time.
Clause 27. The system of any of Clauses 21-26, further comprising a user interface in communication with the resource allocation control unit, wherein the resource allocation control unit outputs a resource allocation signal including one or more resource recommendations to the user interface, and wherein the user interface shows the one or more resource recommendations on a display of the user interface.
Clause 28. The system of any of Clauses 21-27, wherein the enclosed space comprises an internal cabin of a vehicle.
Clause 29. The system of Clause 28, wherein the one or more sensors are associated with one or more seats within the internal cabin.
Clause 30. The system of any of Clauses 21-29, wherein the one or more triggering events comprise one or both of a cough or a sneeze.
Clause 31. The system of any of Clauses 21-30, wherein the one or more sensors comprise one or more of at least one microphone, at least one camera, at least one particulate sensor, or at least one pressure sensor.
Clause 32. A method for allocating sanitizing resources within an enclosed space, the method comprising:
   generating, by a resource allocation control unit, one or more resource recommendations regarding one or more ultraviolet (UV) lamps for the enclosed space based on one or more triggering events of one or more actions detected by one or more sensors within the enclosed space.
Clause 33. The method of Clause 32, further comprising:
   receiving, by the resource allocation control unit, one or more action detection signals indicative of the one or more actions from the one or more sensors; and
   determining, by the resource allocation control unit, the one or more triggering events from the one or more action detection signals.
Clause 34. The method of Clauses 32 or 33, further comprising storing, by the resource allocation control unit, data regarding the triggering events over a predetermined time in the memory.
Clause 35. The method of Clause 34, wherein the data comprises locations of the triggering events.
Clause 36. The method of Clause 35, wherein the data further comprises dates of the triggering events.
Clause 37. The method of any of Clauses 32-36, further comprising generating, by the resource allocation control unit, an event map that shows the areas of the triggering events over a predetermined period of time.
Clause 38. The method of any of Clauses 32-36, further comprising:
   outputting, by the resource allocation control unit, a resource allocation signal including one or more resource recommendations to a user interface; and
   showing, on a display of the user interface, the one or more resource recommendations.
Clause 39. The method of any of Clauses 32-38, wherein the enclosed space comprises an internal cabin of a vehicle.
Clause 40. The method of any of Clauses 32-39, wherein the one or more triggering events comprise one or both of a cough or a sneeze.

As described herein, embodiments of the present disclosure provide systems and methods for effectively and efficiently operating UV light emitters. Further, embodiments of the present disclosure provide systems and methods for operating UV light emitters in environments in which resources are limited. Additionally, embodiments of the present disclosure provide systems and methods for allocating sanitizing resources within an environment, such an internal cabin of a vehicle.

While various spatial and directional terms, such as top, bottom, lower, mid, lateral, horizontal, vertical, front and the like can be used to describe embodiments of the present disclosure, it is understood that such terms are merely used with respect to the orientations shown in the drawings. The orientations can be inverted, rotated, or otherwise changed, such that an upper portion is a lower portion, and vice versa, horizontal becomes vertical, and the like.

As used herein, a structure, limitation, or element that is "configured to" perform a task or operation is particularly structurally formed, constructed, or adapted in a manner corresponding to the task or operation. For purposes of clarity and the avoidance of doubt, an object that is merely capable of being modified to perform the task or operation is not "configured to" perform the task or operation as used herein.

It is to be understood that the above description is intended to be illustrative, and not restrictive. For example, the above-described embodiments (and/or aspects thereof) can be used in combination with each other. In addition, many modifications can be made to adapt a particular situation or material to the teachings of the various embodiments of the disclosure without departing from their scope. While the dimensions and types of materials described herein are intended to define the parameters of the various embodiments of the disclosure, the embodiments are by no means limiting and are exemplary embodiments. Many other embodiments will be apparent to those of skill in the art upon reviewing the above description. The scope of the various embodiments of the disclosure should, therefore, be determined with reference to the appended claims, along with the full scope of equivalents to which such claims are entitled. In the appended claims and the detailed description herein, the terms "including" and "in which" are used as the plain-English equivalents of the respective terms "comprising" and "wherein." Moreover, the terms "first," "second," and "third," etc. are used merely as labels, and are not intended to impose numerical requirements on their objects.

This written description uses examples to disclose the various embodiments of the disclosure, including the best mode, and also to enable any person skilled in the art to practice the various embodiments of the disclosure, including making and using any devices or systems and performing any incorporated methods. The patentable scope of the various embodiments of the disclosure is defined by the claims, and can include other examples that occur to those skilled in the art. Such other examples are intended to be within the scope of the claims if the examples have structural elements that do not differ from the literal language of the claims, or if the examples include equivalent structural elements with insubstantial differences from the literal language of the claims.

## Claims

1. A system (100) for sanitizing an enclosed space (104), the system (100) comprising:
one or more sensors (120) within the enclosed space (104), wherein the one or more sensors (120) are configured to detect one or more actions within the enclosed space (104) and output one or more action detection signals (130) indicative of the one or more actions;
one or more ultraviolet (UV) lamps within the enclosed space (104), wherein the one or more UV lamps (110) are configured to emit UV light (112) in relation to one or more portions of the enclosed space (104); and
a sanitizing control unit (116) in communication with the one or more sensors (120) and the one or more UV lamps (110), wherein the sanitizing control unit (116) is configured to receive the one or more action detection signals (130) from the one or more sensors (120), and wherein the sanitizing control unit (116) is configured to modify operation of the one or more UV lamps (110) in response to the one or more actions being a triggering event.

2. The system (100) of claim 1, wherein the enclosed space (104) comprises an internal cabin of a vehicle.

3. The system (100) of claim 2, wherein the one or more sensors (120) are associated with one or more seats within the internal cabin.

4. The system (100) of any of claims 1 to 3, wherein the UV lamps (110) are configured to emit the UV light (112) within a far UV spectrum.

5. The system (100) of any of claims 1 to 4, wherein the triggering event comprises one or both of a cough or a sneeze.

6. The system (100) of any of claims 1 to 5, wherein the one or more sensors (120) comprise one or more of at least one microphone (122), at least one camera (124), at least one particulate sensor, or at least one pressure sensor.

7. The system (100) of any of claims 1 to 6, wherein the one or more sensors (120) comprises at least one microphone (122), at least one camera (124), at least one particulate sensor, and at least one pressure sensor.

8. The system (100) of any of claims 1 to 7, wherein the sanitizing control unit (116) is configured to modify operation of the one or more UV lamps (110) in response to the one or more actions being a triggering event by activating the one or more UV lamps (110) to emit the UV light (112) onto the one or more surfaces (102).

9. The system (100) of any of claims 1 to 8, wherein the sanitizing control unit (116) is configured to modify operation of the one or more UV lamps (110) in response to the one or more actions being a triggering event by increasing an intensity of the UV light (112).

10. The system (100) of any of claims 1 to 9, wherein the one or more sensors (120) and the one or more UV lamps (110) are integrated in a common housing (150).

11. The system (100) of any of claims 1 to 10, further comprising a resource allocation control unit (132) in communication with the one or more sensors (120), wherein the resource allocation control unit (132) is configured to generate one or more resource recommendations regarding the one or more UV lamps (110) for the enclosed space (104) based on one or more of the triggering events.

12. The system (100) of claim 11, wherein the resource allocation control unit (132) is configured to receive the one or more action detection signals (130) from the one or more sensors (120), and wherein the resource allocation control unit (132) is configured to determine the one or more triggering events from the one or more action detection signals (130).

13. The system (100) of any of the claims 11 or 12 wherein the resource allocation control unit (132) stores data regarding the triggering events over a predetermined time in the memory (134).

14. The system (100) of any of the claims 11-13 wherein the resource allocation control unit (132) is further configured to generate an event map (136) that shows the areas of the triggering events over a predetermined period of time.

15. The system (100) of any of the claims 11-14 wherein the one or more sensors (120) comprise one or more of at least one microphone (122), at least one camera (124), at least one particulate sensor, or at least one pressure sensor.
